# EUROPÄISCHE PATENTSCHRIFT

(11) **EP 0 375 990 B1**
(45) Veröffentlichungstag und Bekanntmachung des Hinweises auf die Patenterteilung: **06.04.1994**
(21) Anmeldenummer: 89122183.0
(22) Anmeldetag: 01.12.1989
(51) Int. Cl.: C09B 1/503, C07C 225/36

(54) **Verfahren zur Herstellung von 1-Amino-2-brom-4-hydroxy-anthrachinon**
Process for the preparation of 1-amino-2-bromo-4-hydroxy-anthraquinone
Procédé de préparation de la 1-amino-2-bromo-4-hydroxy-anthraquinone

(30) Priorität: 14.12.1988 DE 3841988
(43) Veröffentlichungstag der Anmeldung: 04.07.1990
(73) Patentinhaber: BASF Aktiengesellschaft, 67063 Ludwigshafen (DE)
(72) Erfinder: Epple, Gerhard, Dr., D-6719 Weisenheim (DE)

(56) Entgegenhaltungen:
- FR-A- 1 352 537
- US-A- 3 086 977
- CHEMICAL ABSTRACTS, Band 83, 1975, Seite 123, Zusammenfassung Nr. 81233s, Columbus, Ohio, US; & JP-A-75 18 526 (NIPPON KAYAKU CO., LTD) 27-02-1975

## Beschreibung

Die vorliegende Erfindung betrifft ein neues Verfahren zur Herstellung von 1-Amino-2-brom-4-hydroxy-anthrachinon (I) 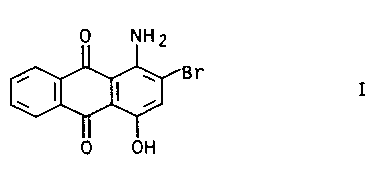
durch Bromierung von 1-Amino-4-hydroxy-anthrachinon (II) 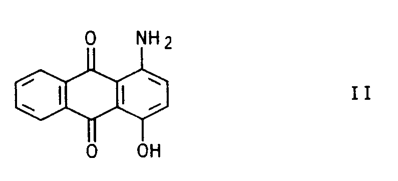
In der DE-A 27 13 575 wird die Herstellung von (I) ausgehend von 1-Amino-anthrachinon beschrieben, wobei man zunächst die Ausgangsverbindung in schwefelsaurem Medium mit Brom in das 2, 4-Dibrom-anthrachinon überführt, welches dann mit Borsäure hydrolysiert wird.

Nach der Lehre der GB-A 1 239 778 wird die Halogenierung von Anthrachinonderivaten in Gegenwart von Mischungen aus einwertigen Alkoholen und inerten organischen Flüssigkeiten vorgenommen. Eigene Versuche haben jedoch gezeigt, daß die Bromierung unter diesen Bedingungen nur als Nebenreaktion abläuft.

Aus der US-A-3 086 977 ist die Halogenierung von (II) bei Raumtemperatur in Pyridin bekannt. Auf diese Weise lassen sich jedoch nur sehr unsaubere Produkte (I) in zudem unzureichender Ausbeute erhalten.

Die JP-A 042 631 betrifft die Halogenierung der mineralsauren Salze von (II) in inerten organischen Lösungsmitteln. Dieses Verfahren ist jedoch weniger selektiv bezüglich der Monobromierung und liefert, wie festgestellt wurde, (I) mit geringerer Reinheit und nur in Ausbeuten von 70 bis 80 %.

Da die bisher bekannten Verfahren in Bezug auf die Reinheit und die Ausbeuten unbefriedigend oder verfahrenstechnisch aufwendig sind, lag der Erfindung die Aufgabe zugrunde, diesen Mängeln abzuhelfen.

Demgemäß wurde ein neues Verfahren zur Herstellung von 1-Amino-2-brom-4-hydroxy-anthrachinon (I) durch Bromierung von 1-Amino-4-hydroxy-anthrachinon (II) gefunden, welches dadurch gekennzeichnet ist, daß man die Bromierung in einer inerten aprotischen organischen Flüssigkeit bei 100 bis 180°C durchführt.

1-Amino-4-hydroxy-anthrachinon (II) ist bekannt und nach bekannten Verfahren erhältlich, z.B. durch Umsetzung von 1, 4-Diaminoanthrachinon mit Mangandioxid in Schwefelsäure. Für die meisten Zwecke genügt es, (II) in technischer Qualität (etwa 95 gew.%iger Reinheit) einzusetzen.

Die Ausgangsverbindung (II) wird in der inerten organischen Flüssigkeit suspendiert. Als inerte aprotische organische Flüssigkeiten eignen sich solche Verbindungen, die unter den Reaktionsbedingungen selber nur in unbedeutenden Mengen reagieren, darunter vorzugsweise mit elektronenziehenden Gruppen substituierte Monoaromaten wie beispielsweise Nitrobenzol, o-Dichlorbenzol, Trichlorbenzol oder Benzoesäuremethylester.

Die Menge an inerter organischer Flüssigkeit beträgt in der Regel 4 bis 12, vorzugsweise 6 bis 9 kg pro kg (II).

Als Bromierungsmittel verwendet man Phosphortribromid, -oxybromid oder -pentabromid oder vor allem elementares Brom. Man setzt diese Verbindungen im allgemeinen in stöchiometrischen Mengen ein, jedoch kann es sich auch empfehlen, das Bromierungsmittel in einem überschuß von 20 äqu.-% zu verwenden. Ein größerer überschuß ist im Hinblick auf die Aufarbeitung des Reaktionsgemisches weniger ratsam.

Im allgemeinen führt man die Reaktion bei 100 bis 180, vorzugsweise bei 120 bis 140°C durch.

Verfahrenstechnisch geht man im allgemeinen so vor, daß man eine Suspension von (II) in der organischen Phase vorlegt und das Bromierungsmittel bei der Reaktionstemperatur zugibt. Gegebenenfalls kann man die Temperatur im Laufe der fortschreitenden Reaktion noch erhöhen.

Zweckmäßigerweise führt man die Reaktion bei Normaldruck durch, aber es ist auch möglich, in einem geschlossenen System unter Eigendruck zu arbeiten.

Man kann die Umsetzung nach den üblichen Techniken auch kontinuierlich, z.B. in einer Rührkesselkaskade, vornehmen.

Der Reaktionsverlauf wird zweckmäßigerweise dünnschichtchromatographisch überwacht. Wenn die Ausgangsverbindung (II) nur noch in Spuren vorhanden ist, kühlt man das Reaktionsgemisch zweckmäßigerweise auf ca. 25°C ab, wonach man wie üblich auf das Verfahrensprodukt aufarbeitet, z.B. indem man es mit Methanol aus der organischen Lösung ausfällt. Der ausfallende Feststoff wird abfiltriert und mit Methanol und Wasser gewaschen. Er enthält in der Regel 90 bis 98 Gew.% (I). Die Reinheit kann nach den üblichen Techniken chromatographisch-photometrisch bestimmt werden.

1-Amino-2-brom-4-hydroxy-anthrachinon (I) ist ein wichtiges Zwischenprodukt bei der Herstellung von Anthrachinonfeststoffen, wie z.B. Disperse Red 60 und Disperse Red 91.

Das erfindungsgemäße Verfahren liefert (I) in hervorragender Reinheit und Ausbeute.

### Beispiel 1

30 g 1-Amino-4-hydroxy-anthrachinon (95 gew. -%ig) wurden in 250 g Nitrobenzol suspendiert und auf 100°C erwärmt. Nachdem 22 g Brom innerhalb von 15 min zugetropft worden waren, wurde die Temperatur des Reaktionsgemisches während einer Zeitspanne von einer Stunde auf 130 bis 140°C erhöht. Diese Temperatur wurde noch drei Stunden lang gehalten, und dann wurde das Reaktionsgemisch auf 25°C abgekühlt und mit 150 g Methanol versetzt. Das ausgefallene Reaktionsprodukt wurde abfiltriert, mit 150 g Methanol und dann mit Wasser gewaschen und anschließend getrocknet.

Man erhielt 38,6 g Produkt mit einem Gehalt an (I) von 97,1 %, was einer Ausbeute von 98, 9 % entspricht.

### Beispiel 2

30 g 1-Amino-4-hydroxy-anthrachinon (95 gew.%ig) wurden in 250 g Benzoesäuremethylester suspendiert und auf 110 bis 120°C erwärmt. Zu dieser Suspension wurden innerhalb von 15 min 22 g Brom getropft.

Anschließend wurde das Reaktionsgemisch auf 130 bis 140°C erwärmt und 3 Stunden lang bei dieser Temperatur gehalten. Danach wurde auf 25°C abgekühlt und mit 250 g Methanol versetzt. Das ausgefallene Reaktionsprodukt wurde abfiltriert, zunächst mit 250 g Methanol, dann mit Wasser gewaschen und getrocknet.

Man erhielt 36, 9 g Produkt mit einem Gehalt an (I) von 98, 2 %, was einer Ausbeute von 95, 5 % entspricht.

## Patentansprüche

1. Verfahren zur Herstellung von 1-Amino-2-brom-4-hydroxy-anthrachinon (I) 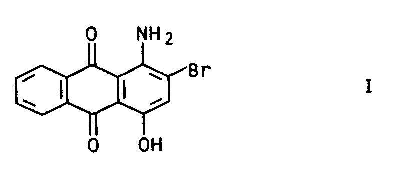 durch Bromierung von 1-Amino-4-hydroxy-anthrachinon (II) 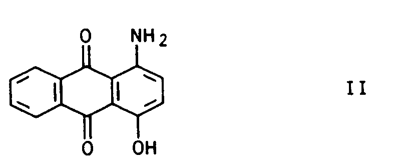 dadurch gekennzeichnet, daß man die Bromierung in einer inerten aprotischen organischen Flüssigkeit bei 100 bis 180°C durchführt.

2. Verfahren gemäß Anspruch 1, dadurch gekennzeichnet, daß man als inerte aprotische organische Flüssigkeiten Nitrobenzol, o-Dichlorbenzol, Trichlorbenzol oder Benzoesäuremethylester verwendet.

## Claims

1. A process for preparing 1-amino-2-bromo-4-hydroxyanthraquinone (I) 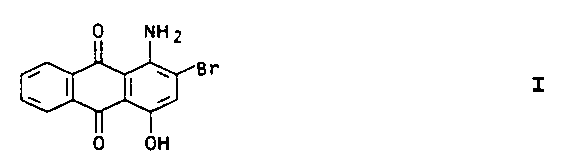 by bromination of 1-amino-4-hydroxyanthraquinone (II) 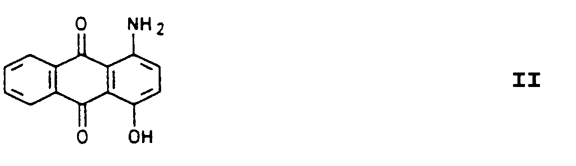 characterized in that the bromination is carried out at from 100 to 180°C in an inert aprotic organic liquid.

2. A process as claimed in claim 1, characterized in that the inert aprotic organic liquid used is nitrobenzene, o-dichlorobenzene, trichlorobenzene or methyl benzoate.

## Revendications

1. Procédé de préparation de 1-amino-2-bromo-4-hydroxyanthraquinone (I) 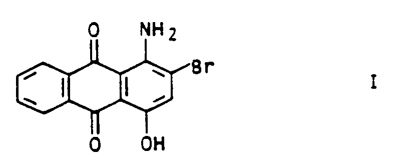 par bromation de 1-amino-4-hydroxyanthraquinone (II) 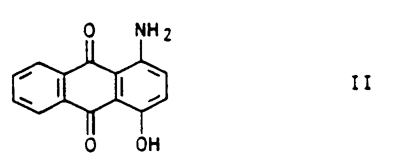 caractérise en ce que l'on effectue la bromation dans un liquide organique aprotique inerte à une température comprise entre 100 et 180°C.

2. Procédé selon la revendication 1, caractérisé en ce que l'on utilise comme liquides organiques aprotiques inertes les nitrobenzène, o-dichlorobenzène, trichlorobenzène ou benzoate de méthyle.
